(19) Europäisches Patentamt
European Patent Office
Office européen des brevets

(11) Veröffentlichungsnummer: **0 392 059 B1**

(12) **EUROPÄISCHE PATENTSCHRIFT**

(45) Veröffentlichungstag der Patentschrift: **15.09.93**

(51) Int. Cl.$^5$: **A61K 31/13**, C07C 211/38

(21) Anmeldenummer: **89106657.3**

(22) Anmeldetag: **14.04.89**

Die Akte enthält technische Angaben, die nach dem Eingang der Anmeldung eingereicht wurden und die nicht in dieser Patentschrift enthalten sind.

# (54) Verwendung von Adamantan-Derivaten zur Prävention und Behandlung der cerebralen Ischämie.

(43) Veröffentlichungstag der Anmeldung:
**17.10.90 Patentblatt 90/42**

(45) Bekanntmachung des Hinweises auf die Patenterteilung:
**15.09.93 Patentblatt 93/37**

(84) Benannte Vertragsstaaten:
**AT BE CH DE ES FR GB GR IT LI LU NL SE**

(56) Entgegenhaltungen:
**EP-A- 0 293 974**
**US-A- 3 450 761**

**RÖMPP CHEMIE LEXIKON, 9. Auflage, 1989, Seite 141&NUM;**

**W. Fleischhacker et al., PROG. NEURO-PSYCHPHARMACOL. & BIOL.-PSYCIATRY, 1986, Band 10, Nr. 1; Seiten 87-93&NUM;**

**J. PHARMACOL., Band 13, Nr. 1, 1982, Paris (FR); R. MARCY et al., Seiten 163-164&NUM;**

**JAPAN. J. PHARMACOL., Band 39, NR. 4, 1985, Kyoto (JP); Y.MIURA et al., Seiten 443-451&NUM;**

(73) Patentinhaber: **Merz & Co. GmbH & Co.**
**Eckenheimer Landstrasse 100-104**
**D-60318 Frankfurt(DE)**

(72) Erfinder: **Bormann, Joachim, Dr.**
**Stalburgstrasse 36**
**D-6000 Frankfurt am Main 1(DE)**
Erfinder: **Gold, Markus R., Dr.**
**Kranichstrasse 9**
**D-6085 Nauheim(DE)**
Erfinder: **Schatton, Wolfgang, Dr.**
**Dörnweg 11**
**D-6236 Eschborn(DE)**

(74) Vertreter: **Beil, Hans Chr., Dr. et al**
**BEIL, WOLFF & BEIL, Rechtsanwälte, Postfach 80 01 40**
**D-65901 Frankfurt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

ARZNEIMITTELFORSCHUNG, Band 32, Nr. 10, 1982, Aulendorf (DE); F.O.MILTNER, Seiten 1268-1270, 1271-1273&NUM;

BRITISH MEDICAL JOURNAL, Band 3, Nr. 5874, 04 August 1973, London (GB); A.M.HAMOEN, Seiten 272-273&NUM;

NO SHINKEI GEKA, Band 12, Nr. 1, 1984, Tokyo (JP); H.KINOMOTA et al., Seiten 37-45&NUM;

DIALOG INFORMATION SERVICE, File 72, (Embase) Acc. Nr. 5254914 (Embase Nr. 83005619)&NUM;

THE MERCK MANUAL OF DIAGNOSIS & THERAPY, R. Berkow, MD Ed., 15.ausgabe, 1987, Merck & Co. Inc., Rahway, NJ (US); Seiten 1336-1340&NUM;

SBORNIK VISOKE SKOLY CHEMICKOTECHNOLOGICKE V PRAZE, Technologie Paliv, Band D 29, 1973, Praha (CS); J. BURKHARD et al., Seiten 91-97&NUM;

NAUNYN-SCHMIEDEBERGS ARCH. PHARMACOL., Band 332, Nr. 1, 1986, Berlin (DE); B.S.MELDRUM et al., Seiten 93-97&NUM;

POL. J. PHARMCOLI. PHARM., Band 35, Nr. 6, 1983, Warszawa (PL); E.CHOJNACKA-WOJCIK et al., Seiten 511-515&NUM;

## Beschreibung

Die vorliegende Erfindung betrifft die Verwendung von Adamantanderivaten der allgemeinen Formel

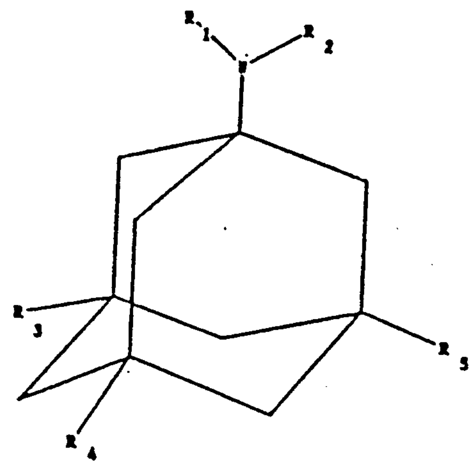

(I)

worin $R_1$ und $R_2$ gleich oder verschieden sind und Wasserstoff oder geradkettige oder verzweigte Alkylgruppen mit 1 bis 6 C-Atomen bedeuten oder zusammengenommen mit N eine heterocyclische Gruppe mit 5 oder 6 C-Ringgliedern darstellen, $R_3$ und $R_4$ jeweils gleich oder verschieden sind und ausgewählt sind aus Wasserstoff, einem geradkettigen oder verzweigten Alkylrest mit 1 bis 6 C-Atomen, einem Cycloalkylrest mit 5 oder 6 C-Atomen, dem Phenylrest,
und worin $R_5$ Wasserstoff oder einen geradkettigen oder verzweigten $C_1$-$C_6$-Alkylrest darstellt.

Hierbei bedeuten verzweigte oder geradketige $C_1$-$C_6$-Alkylreste Methyl, Ethyl, iso- und n-Propyl, n- und iso-Butyl, n-Pentyl und n-Hexyl und Isomere hiervon.

Die Verbindungen eignen sich zur Behandlung der Schädigung von Neuronen infolge eines exzessiven Calciumüberschusses über NMDA-Rezeptorkanäle, insbesondere nach einer cerebralen Ischämie.

Die Erfindung umfasst auch die pharmazeutisch verträglichen Säureadditionssalze der obengenannten Verbindungen.

Gewisse 1-Amino-adamantane der Formel (I) sind an sich bekannt. So ist z.B. 1-Amino-3,5-dimethyladamantan Gegenstand der DE-PS 22 19 256 sowie der DE-PS 28 56 393.

Einige 3,5-disubstituierte 1-Amino-adamantane der Formel (I) sind in der US-PS 4 122 193 beschrieben. 1-Amino-3-ethyl-adamantan ist in der DE-PS 22 32 735 beschrieben.

Allgemein werden Verbindungen der Formel (I) hergestellt durch Alkylierung von Halogen-adamantan, vorzugsweise Brom- oder Chlor-adamantan. Nachfolgende weitere Halogenierung und Alkylierung liefert die einzelnen di- bzw. trisubstituierten Adamantane.

Die Einführung der Aminofunktion erfolgt entweder durch Oxidation mit Chromtrioxid und Bromierung mit HBr oder Bromierung mit Brom und Umsetzung mit Formamid und anschliessender Hydrolyse. Die Alkylierung der Aminofunktion kann nach allgemein bekannten Methoden durchgeführt werden. So lässt sich beispielsweise die Methylierung durch Reaktion mit Chlorameisensäuremethylester und nachfolgende Reduktion durchführen. Die Ethylgruppe kann durch Reduktion des entsprechenden Acetamids eingeführt werden.

Die Aminierung kann auch gemäss der US-A- 4 122 193 durch Umsetzung der entsprechenden 1-Halogen-3,5- oder -7-substituierten Verbindungen mit einem Harnstoffderivat der Formel

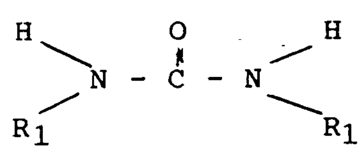

und, falls gewünscht, weiterer Alkylierung der Aminofunktion erfolgen.

Die Herstellung der Verbindungen gemäss Formel (I) ist im nachfolgenden Reaktionsschema dargestellt:

Reaktionsschema

Ad = Adamantylrest
Hal = Cl, Br

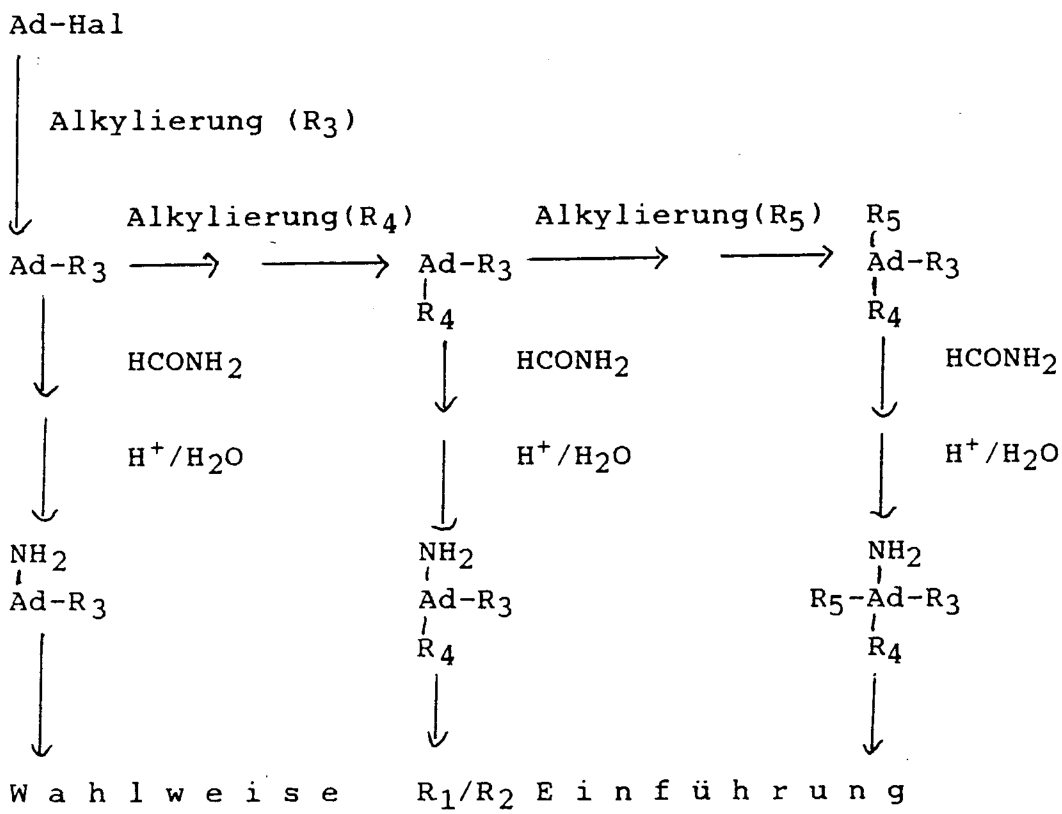


Die Alkylierung der Halogen-adamantane kann z.B. durch Friedel-Crafts-Reaktion (Einführung des Phenylrestes) bzw. durch Reaktion mit Vinylidenchlorid, anschliessender Reduktion und geeigneter Wittig-Reaktion der Aldehyde und deren Hydrierung oder durch Einschub von Ethylen und nachfolgender Alkylierung mit den entsprechenden Cupraten oder durch Einschub von Ethylen und Reduktion der Halogen-Alkyl-Adamantane oder durch Acylierung mit $CO_2$ und Reduktion der Carbonsäuren in an sich bekannter Weise erfolgen.

Die aus den obengenannten Druckschriften bekannten Verbindungen gemäss Formel (I) werden bislang als Mittel zur Behandlung von Morbus Parkinson und parkinsonähnlichen Erkrankungen verwendet. Ihre Wirkungsweise wird auf eine dopaminerge Beeinflussung des ZNS zurückgeführt, vermittelt entweder durch vermehrte Freisetzung oder durch Aufnahmehemmung der Transmittersubstanz Dopamin. Dadurch wird das Ungleichgewicht im Dopamin/Acetylcholinsystem aufgehoben.

In J.Pharmacol., 13, 163-164, 1982 wird der Effekt von dopaminergen Stimulantien, u.a. von 1-Amino-adamantan, auf das Koma-EEG untersucht. Es wird ferner gezeigt, daß Haloperidol die durch das Adamantan bewirkte Wiederherstellung von gestörter EEG-Aktivität aufhebt, da Haloperidol ein Dopamin-Antagonist ist.

In Japan J. Pharmacol. 39, 443-451, 1985, werden ebenfalls dopaminerge Substanzen untersucht, die akut zu einer EEG-Aktivierung bei ponsischämischen Ratten führen.

Ferner wird in Arzneimittelforschung 32, 1268-1273, 1982, die Wirkung von 1-Amino-adamantan bei Koma-Patienten untersucht und die Wirkung dieser Substanz auf den Katecholaminstoffwechsel beschrieben.

In der Veröffentlichung "British Medical Journal, 3, 272-273, 1973, wird das akute Verschwinden charakteristischer EEG-Veränderungen nach L-Dopa- und Amantadin-Therapie bei Patienten mit Jakob-Creutzfeldt-Disease erläutert, wobei die Wirksamkeit der Verbindungen auf deren dopaminergen Einfluß

zurückzuführen ist.

Die US-A 3 450 761 offenbart Amino-adamantane, u.a. 1-Amino-3-ethyl-5,7-dimethyl-adamantan, welche eine antivirale Wirkung aufweisen.

Im Gegensatz zu dieser Art von Erkrankungen liegt bei der cerebralen Ischämie eine pathophysiologische Situation vor, in der die neuronalen Erregungsmechanismen aus dem Gleichgewicht geraten. Dabei führt der exzessive Einstrom von Calcium durch N-Methyl-D-Aspartat(NMDA-)Rezeptorkanäle letztlich zur Zerstörung von Nervenzellen bestimmter Hirnareale (Rothmann & Olney, Trens Neurosci 10, 1987, 299ff). Für die Behandlung bzw. Behebung dieser pathologischen Situation scheint daher ein bezüglich der NMDA-Rezeptorkanäle antagonistisches Eingreifen erforderlich (Kemp et al., Trends Pharmacol., Sci. 8, 1987, 414ff).

Ein solches Eingreifen kann beispielsweise mit substituierten Fluor- und Hydroxyderivaten von Dibenzo-[a,d]-cyclohepten-5,10-imin erfolgen, die in der EP-A 0 264 183 beschrieben sind.

Diese heterocyclisch-aromatischen Verbindungen sind lipophiler Art und weisen NMDA-Rezeptorkanal-antagonistische sowie antikonvulsive Eigenschaften auf. Sie werden nach einem relativ aufwendigen Verfahren hergestellt, wobei Enantiomerenmischungen anfallen, die in die einzelnen optischen Antipoden aufgetrennt werden können.

Der vorliegenden Erfindung liegt die Aufgabe zugrunde, chemisch einfach zugängliche Verbindungen mit einer NMDA-Rezeptorkanal-antagonistischen und einer antikonvulsiven Wirkung bereitzustellen zur Verwendung bei der Prävention und Behandlung der cerebralen Ischämie.

Diese Aufgabe wird erfindungsgemäss gelöst durch die Verwendung der 1-Amino-adamantane der Formel (1),

Überraschend wurde gefunden, dass bei Verwendung dieser Verbindungen die Schädigung von Hirnzellen nach einer Ischämie verhindert werden kann. Die Adamantanderivate der Formel (I) eignen sich daher zur Prävention und zur Therapie cerebraler Ischämien nach Schlaganfall, Herzoperationen, Herzstillstand, Subarachnoidalblutungen, transienter cerebralischämischer Attacken, perinataler Asphyxie, Anoxie, Hypoglykämie, Apnoe und Morbus Alzheimer.

Beispiele erfindungsgemässer Verbindungen sind:

1-Amino-adamantan
1-Amino-3-phenyl-adamantan
1-Amino-methyl-adamantan
1-Amino-3,5-dimethyl-adamantan (Testverb.Nr. 1)
1-Amino-3-ethyl-adamantan (Testverbindung Nr. 2)
1-Amino-3-isopropyl-adamantan (Testverbindung Nr. 3)
1-Amino-3-n-butyl-adamantan
1-Amino-3,5-diethyl-adamantan (Testverbindung Nr. 4)
1-Amino-3,5-diisopropyl-adamantan
1-Amino-3,5-di-n-butyl-adamantan
1-Amino-3-methyl-5-ethyl-adamantan
1-N-Methylamino-3,5-dimethyl-adamantan(Testverb. Nr.5)
1-N-Ethyl-1-amino-3,5-dimethyl-adamantan(Testverb.Nr. 6)
1-N-Isopropyl-amino-3,5-dimethyl-adamantan
1-N,N-Dimethyl-amino-3,5-dimethyl-adamantan
1-N-Methyl-N-isopropyl-amino-3-methyl-5-ethyl-adamantan
1-Amino-3-butyl-5-phenyl-adamantan
1-Amino-3-pentyl-adamantan
1-Amino-3,5-dipentyl-adamantan
1-Amino-3-pentyl-5-hexyl-adamantan
1-Amino-3-pentyl-5-cyclohexyl-adamantan
1-Amino-3-pentyl-5-phenyl-adamantan
1-Amino-3-hexyl-adamantan
1-Amino-3,5-dihexyl-adamantan
1-Amino-3-hexyl-5-cyclohexyl-adamantan
1-Amino-3-hexyl-5-phenyl-adamantan
1-Amino-3-cyclohexyl-adamantan (Testverbindung Nr. 7)
1-Amino-3,5-dicyclohexyl-adamantan
1-Amino-3-cyclohexyl-5-phenyl-adamantan
1-Amino-3,5-diphenyl-adamantan
1-Amino-3,5,7-trimethyl-adamantan

1-Amino-3,5-dimethyl-7-ethyl-adamantan (Testverb. Nr. 8)
1-Amino-3,5-diethyl-7-methyl-adamantan,
deren N-Methyl-, N,N-Dimethyl-, N-Ethyl-, N-Propyl-,
1-N-Pyrrolidino- und 1-N-Piperidino-Derivate,
1-Amino-3-methyl-5-propyl-adamantan
1-Amino-3-methyl-5-butyl-adamantan
1-Amino-3-methyl-5-pentyl-adamantan
1-Amino-3-methyl-5-hexyl-adamantan
1-Amino-3-methyl-5-cyclohexyl-adamantan
1-Amino-3-methyl-5-phenyl-adamantan
1-Amino-3-ethyl-5-propyl-adamantan
1-Amino-3-ethyl-5-butyl-adamantan
1-Amino-3-ethyl-5-pentyl-adamantan
1-Amino-3-ethyl-5-hexyl-adamantan
1-Amino-3-ethyl-5-cyclohexyl-adamantan
1-Amino-3-ethyl-5-phenyl-adamantan
1-Amino-3-propyl-5-butyl-adamantan
1-Amino-3-propyl-5-pentyl-adamantan
1-Amino-3-propyl-5-hexyl-adamantan
1-Amino-3-propyl-5-cyclohexyl-adamantan
1-Amino-3-propyl-5-phenyl-adamantan
1-Amino-3-butyl-5-pentyl-adamantan
1-Amino-3-butyl-5-hexyl-adamantan
1-Amino-3-butyl-5-cyclohexyl-adamantan
sowie deren Säureadditionsverbindungen.

Bevorzugte Verbindungen der Formel (I) sind jene, worin $R_1$ und $R_2$ Wasserstoff bedeuten, wie z.B. 1-Amino-3-ethyl-5,7-dimethyl-adamantan, sowie Verbindungen, worin $R_1$, $R_2$, $R_4$ und $R_5$ Wasserstoff bedeuten, wie z.B. 1-Amino-3-cyclohexyl-adamantanund 1-Amino-3-ethyl-adamantan.

Weiterhin bevorzugte Verbindungen sind solche, worin $R_1$, $R_2$ und $R_5$ Wasserstoff bedeuten, wie z.B. 1-Amino-3-methyl-5-propyl- oder -5-butyl-adamantan, 1-Amino-3-methyl-5-hexyl-bzw. -cyclohexyl-adamantan oder 1-Amino-3-methyl-5-phenyladamantan.

Besonders bevorzugte Verbindungen sind 1-Amino-3,5-dimethyl-adamantan, 1-Amino-3,5-diethyl-adamantan, d.h. Verbindungen, worin $R_1$, $R_2$ und $R_5$ Wasserstoff bedeuten, sowie jene, worin $R_1$ und $R_5$ Wasserstoff, $R_2$ Methyl oder Ethyl und $R_3$ und $R_4$ jeweils Methyl bedeuten, wie z.B. 1-N-Methylamino-3,5-dimethyl-adamantan und 1-N-Ethylamino-3,5-dimethyladamantan.

Die Adamantanderivate der Formel (I) können als solche oder in Form ihrer pharmazeutisch verträglichen Säureadditions salze verabreicht werden. Hierzu zählen beispielsweise die Hydrochloride, Hydrobromide, Sulfate, Acetate, Succinate oder Tartrate, Additionsverbindungen mit Fumar-, Malein-, Zitronen- oder Phosphorsäure.

Die Verbindungen der Formel (I) werden in geeigneter Form in Mengen von etwa 0,01 bis 100 mg/kg verabreicht. Geeignete Darreichungsformen sind z.B. Kombinationen des Wirkstoffs mit üblichen pharmazeutischen Trägern und Hilfsmitteln in Form von Tabletten, Dragees, sterilen Lösungen oder Injektionen. Als pharmazeutisch verträgliche Träger können z.B. Lactose, Sucrose, Sorbit, Talcum, Stearinsäure, Magnesiumstearat, Gummiarabicum, Maisstärke oder Cellulose zusammen mit Verdünnungsmitteln wie Wasser, Polyethylenglykol u.ä. verwendet werden. Feste Darreichungsformen werden nach üblichen Methoden hergestellt und können bis zu 50 mg Wirkstoff/Einheit enthalten.

Die Wirksamkeit der Verbindungen der Formel (I) wird anhand der nachfolgenden pharmakologischen Untersuchungen aufgezeigt.

A. Verdrängung der TCP-Bindung

Phencyclidin (PCP), ein bekannter NMDA-Antagonist, bindet an den mit dem NMDA-Rezeptor assoziierten Ionenkanal und blockiert den Ionentransport (Garthwaite & Garthwaite, Neurosci. Lett.83, 1987, 241-246). Ferner gilt als nachgewiesen, dass PCP den Untergang von Nervenzellen als Folge cerebraler Ischämie bei Ratten zu verhindern vermag (Sauer et al., Neurosci. Lett. 91, 1988, 327-332).

Im folgenden wird untersucht, oh die Verbindungen der Formel (I) mit der Bindungsstelle für PCP interagieren. Hierzu Wird $^3H$-TCP, ein Analogon von PCP, eingesetzt.

Eine Membranpräparation vom Rattencortex wird mit $^3$H-TCP, einem Analogon von Phencyclidin (PCP), inkubiert (Quirion & Pert 1982, Eur.J.Pharmaccol. 83:155). Für Testverbindung Nr. 1: 1-Amino 3,5-dimethyladamantan wird die Wechselwirkung mit der TCP-Bindungsstelle im Kompetitionsexperiment ermittelt.

Dabei zeigt sich, dass Testverbindung Nr. 1 in der Lage ist, TCP hochwirksam aus der Bindung zu verdrängen. Der $IC_{50}$-Wert beträgt 89 nM. Daraus folgt, dass Testverbindung Nr. 1 an NMDA-Rezeptorkanäle bindet, und zwar dort, wo der NMDA-Antagonist PCP angreift.

B. Blockierung von NMDA-Rezeptorkanälen

Im folgenden Test wird gezeigt, dass die erfindungsgemässen Verbindungen der Formel (I) ebenso wie PCP den NMDA-Rezeptorkanal blockieren.

Im patch clamp-Experiment wird der Strom durch NMDA-aktivierte Membrankanäle kultivierter Rückenmarksneurone (Maus) gemessen (Hamill et al 1981, Pflügers Arch. 312: 85-100). Dabei wird das Stromsignal der Zelle nach Gabe von 20 $\mu$M NMDA über einen Zeitraum von 20 Sekunden integriert und als Kontrollantwort ($A_c$) gewertet. Bei der sich anschliessenden gemeinsamen Applikation von 20 $\mu$M NMDA und 6 $\mu$M eines Adamantanderivates kann die Stärke der Substanzwirkung als relative Änderung der Kontrollantwort, $A/A_c$, bestimmt werden (A = Testantwort).

Die Ergebnisse sind in nachfolgender Tabelle 1 zusammengefasst:

Tabelle 1

| Verbindung Nr. | $1-A/A_c$ | n |
|---|---|---|
| 1 | 0,66±0,05 | 14 |
| 2 | 0,44±0,08 | 7 |
| 3 | 0,58±0,07 | 7 |
| 4 | 0,50±0,11 | 5 |
| 5 | 0,56±0,07 | 7 |
| 6 | 0,38±0,05 | 7 |
| 7 | 0,25±0,04 | 11 |
| 8 | 0,50±0,03 | 6 |
| PCP | 0,50±0,04 | 7 |
| MK-801 | 0,60±0,05 | 22 |
| Werte sind angegeben als Mittelwert ± S.E.M. | | |

Wie hieraus ersichtlich ist, sind die Amino-adamantanderivate der Formel (I) in der Lage, den NMDA-Rezeptorkanal zu blockieren, wie es auch für PCP (Bertolini et al., Neurosci. Lett. 84, 1988, 351-355) sowie für 5-Methyl-10,11-dihydro-5H-dibenzo[a,d]-cyclohepten-5,10-imin (MK-801) (EP-A 0 264 183) beschrieben wurde.

C. Antikonvulsive Wirkung

Mäuse werden mit 4, 12, 36, 108 und 324 mg/kg der Testsubstanz ip-appliziert (5 Tiere je Dosis). Der supermaximale Elektroschocktest wurde 40 Minuten nach der Substanzgabe angewendet, um den Schutz der Substanz vor Krämpfen zu untersuchen. Die geschützten Tiere werden über alle Dosierungen aufaddiert (Score; Maximum = 25 Tiere).

Die Ergebnisse sind in nachfolgender Tabelle 2 angegeben.

Tabelle 2

| Verbindung Nr. | Antikonvulsive Wirkung (Score) | Mittelwert | $ED_{50}$ (mg/kg) |
|---|---|---|---|
| 1 | 18 | | |
| | 16 | | |
| | 16 | | |
| | 15 | 16,3 | 16 |
| 2 | 15 | | |
| | 14 | | |
| | 12 | 13,7 | 30 |
| 4 | 16 | | |
| | 16 | | |
| | 11 | 14,3 | 24 |
| 5 | 17 | | |
| | 17 | 17,0 | 13 |
| Standards: | | | |
| PCP | 19 | 19,0 | 9 |
| MK-801 | 25 | 25,0 | <1 |

Die $ED_{50}$-Werte sind nach Litchfield und Wilcoxon (1949) geschätzt.

Wie hieraus ersichtlich ist, sind Amino-adamantanderivate der Formel (I) in der Lage, vor elektrisch induzierten Krämpfen zu schützen. Sie sind somit antikonvulsiv wirksam.

D. Korrelation zwischen Kanalblockade und antikonvulsiver Wirkung

Es wird geprüft, ob die Wirkung der getesteten Adamantanderivate 1-8 am NMDA-Rezeptorkanal (in vitro) mit der antikonvulsiven Wirkung (in vivo) im Zusammenhang steht. Dazu wird ein x-y Diagramm der beiden Testparameter erstellt, welches in Abbildung 1 dargestellt ist.

Hieraus kann entnommen werden, dass für die Adamantane der Formel (I) eine Korrelation zwischen der Blockade des NMDA-Rezeptorkanals und der antikonvulsiven Wirkung besteht.

E. Protektion vor cerebraler Ischämie

Bei Ratten werden beide Carotis-Arterien für 10 Minuten verschlossen und im gleichen Zeitraum der Blutdruck durch Blutentnahme auf 60-80 mg Hg abgesenkt (Smith et al. 1984, Acta Neurol. Scand. 69: 385, 401). Die Ischämie wird dann durch öffnen der Karotiden und Reinfusion des entnommenen Blutes beendet. Sieben Tage danach werden die Gehirne der Versuchstiere histologisch auf Zellveränderungen in der CA1-CA4-Region des Hippocampus untersucht und der prozentuale Anteil der Neuronenuntergänge ermittelt. Die Wirkung von Testverbindung Nr. 1 wird nach Einmalgabe von 5 mg/kg und 20 mg/kg ip eine Stunde vor der Ischämie geprüft.

Die Ergebnisse sind in nachfolgender Tabelle 3 zusammengefasst:

Tabelle 3

| Areal | Kontrolle | Testverbindung Nr. 1 | |
|---|---|---|---|
| | | 5 mg/kg (n = 5) | 20 mg/kg (n = 6) |
| CA1 | 80,2±1,5 | 83,0±2,2 | 53,1±6,1** |
| CA3 | 3,6±1,1 | 7,3±1,8 | 2,7±1,0 |
| CA4 | 1,4±0,4 | 3,7±1,7 | 0,6±0,3 |
| Die Werte sind angegeben als Prozent der geschädigten Neuronen ± S.E.M. Signifikanz der Mittelwertdifferenz: | | | |

**p < 0,01 (U-Test)

Hieraus ergibt sich, dass präischämische Applikation von 20 mg/kg der Testverbindung Nr. 1 die postischämischen neuronalen Zellschäden im CA1-Areal des Rattenhippocampus statistisch signifikant reduziert. Unter der Behandlung werden physiologische Parameter (z.B. Blutdruck, Körpertemperatur) nicht verändert. Die Ergebnisse zeigen ferner, dass die Verbindungen gemäss Formel (I) eine neuroprotektive Wirkung bei cerebraler Ischämie haben.

Die Erfindung wird anhand der folgenden Beispiele erläutert.

Beispiel 1

Injektionslösung

Zur Herstellung einer 0,5%igen Lösung werden 0,5% Wirkstoff und 0,8% Natriumchlorid DAB 9 in bidestilliertem Wasser gelöst. Die Lösung wird durch ein Entkeimungsschichtenfilter filtriert, in 2-ml-Ampullen abgefüllt und 20 Minuten bei 120 ° C im Autoklav heiss-sterilisiert.

Beispiel 2

Lösung

1% Wirkstoff wird in demineralisiertem Wasser gelöst. Die Lösung wird vor der Abfüllung filtriert.

Beispiel 3

Tabletten

| 1 Tablette besteht aus: | |
|---|---|
| Wirkstoff | 10,0 mg |
| Milchzucker | 67,5 mg |
| Mikrokristalliner Cellulose | 18,0 mg |
| Talcum | 4,5 mg |
| | 100,0 mg |

Die Substanzen werden gemischt und im Direkttablettierverfahren ohne Granulation zu Tabletten a 100 mg verpresst.

Beispiel 4

Dragees

Es werden, wie unter Tabletten beschrieben, 6 mm Dragee-Kerne mit einem Gewicht von 100 mg hergestellt. Die Hülle wird nach dem Zuckerdragierverfahren aufgetragen: Der Kern wird zunächst mit

Dragiersuspension angedeckt, dann mit Farbsirup gefärbt und schliesslich poliert.

Die Drageehülle besteht aus:

| | |
|---|---|
| Zucker | 65,0 mg |
| Talcum | 39,0 mg |
| Calciumcarbonat | 13,0 mg |
| Gummiarabicum | 6,5 mg |
| Maisstärke | 3,7 mg |
| Schellack | 1,1 mg |
| Polyethylenglykol 6000 | 0,2 mg |
| Magnesia usta | 1,3 mg |
| Farbstoff | 0,2 mg |
| | 130,0 mg |
| Gesamtdragee-Gewicht = 230 mg | |

Beispiel 5

Zur Herstellung einer 0,01%igen Infusionslösung werden 0,01% Wirkstoff und 5% Laevulose in bidestilliertem Wasser gelöst. Die Lösung wird durch Entkeimungsfilter filtriert, in 500 ml Infusionsflaschen abgefüllt und sterilisiert.

Das Beispiel bezieht sich auf 50 mg Wirkstoff pro Einzeldosis.

Beispiel 6

Synthese von 1-Amino-3-isopropyl-adamantan-Hydrochlorid (Testverbindung Nr. 3)

A. Die Darstellung von Adamantancarbonsäuremethylester(I)

1,0 Mol Adamantancarbonsäure werden in 600 ml Methanol gerührt. Unter Eiskühlung werden innerhalb 1h 1,53 Mol Acetylchlorid zugetropft. Das Eisbad wird entfernt und das Reaktionsgemisch auf Raumtemperatur kommen lassen, anschliessend wird 3h unter Rückfluss erhitzt. Das Reaktionsgemisch wird im Vakuum eingeengt und destilliert. (97% Ausbeute)

B. Darstellung von 2-Adamantyl-propan-2-ol(II)

0,5 Mol Magnesiumspäne werden in 50 ml absolutem Ether vorgelegt und 0,5 Mol Methyliodid unter Feuchtigkeitsausschluss so zugetropft, dass der Ether siedet. Anschliessend erwärmt man auf dem Wasserbad bis alles Magnesium gelöst ist.

Zu dieser Lösung tropft man bei Raumtemperatur eine Lösung von 0,2 Mol Adamantancarbonsäuremethylester(I) in absolutem Ether. Nach erfolgter Zugabe erhitzt man 3h zum Rückfluss. Nach dem Abkühlen hydrolysiert man mit Eis und versetzt mit Ammoniumchloridlösung bis der Niederschlag gelöst ist. Die Etherphase wird abgetrennt, die wässrige Phase 2x mit Ether gewaschen und die vereinigten organischen Phasen mit Natriumbicarbonat-Lösung gewaschen, getrocknet und im Vakuum eingeengt. (Ausbeute 93%)

C. Darstellung von Isopropenyl-adamantan(III)

0,25 Mol 2-Adamantyl-propan-2-ol(II) werden in 500 ml Essigsäureanhydrid bei 160 °C 12h gerührt. Anschliessend wird das Reaktionsgemisch auf 1 l Eiswasser gegossen und mit Ether extrahiert. Die vereinigten organischen Phasen werden mit Magnesiumsulfat getrocknet, filtriert und im Vakuum eingeengt. Der Rückstand wird im Vakuum destilliert. (Ausbeute 66%)

D. Darstellung von Isopropyl-adamantan(IV)

0,074 Mol Isopropenyl adamantan(III) werden in 100 ml absolutem Ethanol gelöst. Man gibt 4 g Palladium (5% auf Aktivkohle) zu und hydriert 24 h bei Raumtemperatur unter Rühren. Danach wird vom

Katalysator abfiltriert und das Lösungsmittel im Vakuum entfernt. (Ausbeute 91%)

E. Darstellung von 1-Brom-3-isopropyl-adamantan(V)

0,034 Mol Isopropyl-adamantan(IV) werden mit dem zehnfachen Überschuss an Brom versetzt (0,33 Mol). Man erwärmt langsam und rührt 4h unter Rückfluss. Anschliessend lässt man abkühlen und giesst auf Eiswasser. Das überschüssige Brom wird mit Natriumsulfit zersetzt, bis die wässrige Lösung entfärbt ist. Danach extrahiert man mit Ether, wäscht die vereinigten organischen Phasen mit Natriumbicarbonat-Lösung, trocknet mit Magnesiumsulfat, filtriert und engt im Vakuum ein. Der erhaltene Rückstand wird aus Methanol umkristallisiert. (Ausbeute 83%)

F. Darstellung von 1-Formyl-amido-3-isopropyl-adamantan(VI)

0,028 Mol 1-Brom-3-isopropyl-adamantan(V) werden mit 40 ml Formamid 12 h zum Rückfluss erhitzt. Nach dem Abkühlen wird das Reaktionsgemisch auf Wasser gegossen und mit Dichlormethan extrahiert. Die vereinigten organischen Phasen werden mit Magnesiumsulfat getrocknet, filtriert und im Vakuum eingeengt. (Ausbeute 82%)

G. Darstellung von 1-Amino-3-isopropyl-adamantan-Hydrochlorid

0,023 Mol 1-Formyl-amido-3-isopropyl-adamantan(VI) werden mit 100 ml 15%iger Salzsäure versetzt und 24h zum Sieden erhitzt. Nach dem Abkühlen wird der Niederschlag abfiltriert und aus Isopropanol umkristallisiert. (Ausbeute 57%)

Beispiel 7

Synthese von 1-Amino-3-cyclohexyl-adamantan-Hydrochlorid (Testverbindung Nr. 7)

A. Darstellung von 1-Phenyl-adamantan(I)

0,068 Mol Eisen(III)-chlorid werden in 20 ml absolutem Benzol zum Sieden erhitzt. Unter Rühren werden 0,0186 Mol 1-Brom-adamantan, in 30 ml abs. Benzol gelöst, zugetropft. Danach wird 3 h zum Sieden erhitzt. Das Reaktionsgemisch wird nach dem Abkühlen auf Eis/Salzsäure gegossen, die organische Phase abgetrennt und die wässrige Phase noch zwei Mal mit Benzol extrahiert. Die vereinigten organischen Phasen werden mit Wasser gewaschen, mit Calciumchlorid getrocknet, filtriert und im Vakuum eingeengt. Der Rückstand wird aus Methanol umkristallisiert. (Ausbeute 80%)

B. Darstellung von 1-Hydroxy-3-phenyl-adamantan(II)

Zu einer Lösung aus 0,03 Mol Chromtrioxid in 20 ml Eisessig und 20 ml Essigsäureanhydrid werden bei 0 °C 0,0095 Mol 1-Phenyl-adamantan zugegeben und 24 h bei 4 °C gerührt. Das Reaktionsgemisch wird dann auf Wasser gegossen und drei Mal mit Pentan extrahiert. Die organische Phase wird mit gesättigter Natriumchlorid-Lösung gewaschen, über Magnesiumsulfat getrocknet, filtriert und im Vakuum eingeengt. Der Rückstand wird mit 20 ml 2N NaOH und 50 ml Methanol hydrolysiert. Das Methanol wird anschliessend im Vakuum entfernt und der Rückstand mit Wasser verdünnt. Dann wird drei Mal mit Ether extrahiert. Die organische Phase wird gtrocknet, filtriert und im Vakuum eingeengt. Der Rückstand wird aus Cyclohexan umkristallisiert. (Ausbeute 50%)
Lit.: H.Stetter, M.Schwarz u. A.Hirschhorn, Chem.Ber. (1959), 92,1629-35.

C. Darstellung von 1-Brom-3-phenyl-adamantan(III)

0,03 Mol 3-Phenyl-adamantanol(II) werden mit 100 ml 40%iger HBr in Eisessig 20min bei 60 °C und 30min bei Raumtemperatur gerührt. Das Reaktionsgemisch wird anschliessend mit Wasser verdünnt und mit Ether extrahiert. Die vereinigten organischen Extrakte werden mit Natriumchlorid-Lösung gewaschen, mit Magnesiumsulfat getrocknet, filtriert und im Vakuum eingeengt. Der Rückstand wird aus Methanol umkristallisiert. (Ausbeute 68%)
Lit.: W.Fischer u. C.A.Grog, Helvetica Chim.Acta (1976), 59,1953.

D. Darstellung von 1-Formyl-amido-3-phenyl-adamantan(IV)

0,03 Mol 1-Brom-3-phenyl-adamantan(III) werden mit 50 ml Formamid 12h zum Rückfluss erhitzt. Nach dem Abkühlen giesst man das Reaktionsgemisch auf Wasser und extrahiert mit Dichlormethan. Die vereinigten organischen Phasen werden mit Magnesiumsulfat getrocknet, filtriert ud eingeengt. (Ausbeute 80%)

E. Darstellung von 1-Amino-3-phenyl-adamantan-Hydrochlorid(V)

0,02 Mol 1-Formyl-amido-3-phenyl-adamantan(IV) werden mit 100ml 15%iger Salzsäure 24h zum Sieden erhitzt. Nach dem Abkühlen wird der Niederschlag abfiltriert und aus Isopropanol umkristallisiert. (Ausbeute 60%)

F. Darstellung von 1-Amino-3-cyclohexyl-adamantan(VI)

0,011 Mol 1-Amino-3-phenyl-adamantan(V) werden in 150ml Eisessig gelöst, mit 0,3 g Platinoxid (1% of Aktivkohle) versetzt und bei 3 bar Wasserstoffdruck in einer Parr-Apparatur bei 35 °C hydriert. Anschliessend wird der Katalysator abfiltriert und das Filtrat eingeengt. Der Rückstand wird mit Methanol aufgenommen und das Produkt mit Ether ausgefällt, abgesaugt und getrocknet. (Ausbeute 70%)

Beispiel 8

Synthese von 1-Amino-3,5-dimethyl-7-ethyl-adamantan-Hydrochlorid (Testverbindung Nr. 8)

A. Darstellung von 1-Brom-3,5-dimethyl-adamantan(I)

0,5 Mol 1,3-Dimethyl-adamantan werden mit dem zehnfachen Überschuss an Brom versetzt (5 Mol). Man erwärmt langsam und rührt 4h unter Rückfluss. Anschliessend lässt man abkühlen und giesst auf Eiswasser. Das überschüssige Brom wird mit Natriumsulfit zersetzt, bis die wässrige Lösung entfärbt ist. Danach extrahiert man mit Ether, wäscht die vereinigten organischen Phasen mit Natriumbicarbonat-Lösung, trocknet mit Magnesiumsulfat, filtriert und engt im Vakuum ein. Der erhaltene Rückstand wird aus Methanol umkristallisiert. (Ausbeute 83%)

B. Darstellung von 1-(2-Brom-ethyl)-3,5-dimethyl-adamantan(II)

1,4 Mol 1-Brom-3,5-dimethyl-adamantan(I) werden bei -75 °C in Hexan mit 0,6 Mol Aluminiumbromid versetzt. Danach leitet man 20 bis 30 Minuten Ethylen durch die Lösung, rührt 5min. nach und giesst das Reaktionsgemisch auf Eiswasser. Man extrahiert mit Ether, trocknet die organische Phase und engt diese ein. Der Rückstand wird aus Methanol umkristallisiert. (Ausbeute 48%)

C. Darstellung von 1,3-Dimethyl-5-ethyl-adamantan(III)

0,5 Mol 1-(2-Brom-ethyl)-3,5-dimethyl-adamantan(II) werden in Toluol gelöst und mit 0,55 Mol Natrium-bis(2-methoxyethoxy)-dihydro-aluminat versetzt und 3h zum Sieden erhitzt. Nach dem Hydrolysieren wird die organische Phase abgetrennt, mit Magnesiumsulfat getrocknet und im Vakuum eingeengt. Der Rückstand wird durch Vakuumdestillation gereinigt. (Ausbeute 86%)

D. Darstellung von 1-Brom-3,5-dimethyl-7-ethyl-adamantan(IV)

0,4 Mol 1,3-Dimethyl-5-ethyl-adamantan(III) werden mit dem zehnfachen Überschuss an Brom versetzt (4 Mol). Man erwärmt langsam und rührt 4h unter Rückfluss. Anschliessend lässt man abkühlen und giesst auf Eiswasser. Das überschüssige Brom wird mit Natriumsulfit zersetzt, bis die wässrige Lösung entfärbt ist. Danach extrahiert man mit Ether, wäscht die vereinigten organischen Phasen mit Natriumbicarbonat-Lösung, trocknet mit Magnesiumsulfat, filtriert und engt im Vakuum ein. Der erhaltene Rückstand wird aus Methanol umkristallisiert. (Ausbeute 86%)

E. Darstellung von 1-Formyl-amido-3,5-dimethyl-7-ethyl-adamantan(V)

0,2 Mol 1-Brom-3,5-dimethyl-7-ethyl-adamantan(IV) werden mit 150 ml Formamid 12h zum Rückfluss erhitzt. Nach dem Abkühlen wird das Reaktionsgemisch auf Wasser gegossen und mit Dichlormethan extrahiert. Die vereinigten organischen Phasen werden mit Magnesiumsulfat getrocknet, filtriert und im Vakuum eingeengt. (Ausbeute 82%)

F. Darstellung des 1-Amino-3,5-dimethyl-7-ethyl-adamantan-Hydrochlorids(VI)

0,2 Mol 1-Formyl-amido-3,5-dimethyl-7-ethyl-adamantan(V) werden mit 100 ml 15%iger Salzsäure versetzt und 24h zum Sieden erhitzt. Nach dem Abkühlen wird der Niederschlag abfiltriert und aus Isopropanol umkristallisiert. (Ausbeute 57%)

Beispiel 9

Synthese von 1-N-Methylamino-3,5-dimethyl-adamantan (Testverbindung Nr. 5)

0,1 Mol des entsprechend substituierten Amino-adamantans (1-Amino-3,5-dimethyl-adamantan) werden mit 0,15 Mol Chlorameisensäureethylester und Kaliumcarbonat in Aceton gelöst und 8h zum Rückfluss erhitzt. Nach dem Abkühlen wird filtriert, das Lösungsmittel abgezogen und der Rückstand getrocknet. Das Rohprodukt (0,05 Mol) wird mit 0,1 Mol Natrium-bis-(2-methoxy-ethoxy)-dihydroaluminat in Toluol versetzt und 3h zum Sieden erhitzt. Nach dem Abkühlen wird mit verd. HCl hydrolysiert, die organische Phase getrocknet und eingeengt. Das Rohprodukt wird durch Destillation gereinigt.

Beispiel 10

Synthese von 1-Amino-3-ethyl-5-phenyl-adamantan

A. Darstellung von 1-Brom-3-ethyl-adamantan(I)

0,034 Mol Ethyl-adamantan werden mit dem zehnfachen Überschuss an Brom versetzt (0,33 Mol). Man erwärmt langsam und rührt 4h unter Rückfluss. Anschliessend lässt man abkühlen und giesst auf Eiswasser. Das überschüssige Brom wird mit Natriumsulfit zersetzt, bis die wässrige Lösung entfärbt ist. Danach extrahiert man mit Ether, wäscht die vereinigten organischen phasen mit Natriumbicarbonat-Lösung, trocknet mit Magnesiumsulfat, filtriert und engt im Vakuum ein. Der erhaltene Rückstand wird aus Methanol umkristallisiert. (Ausbeute 83%)

B. Darstellung von 1-Ethyl-3-phenyl-adamantan(II)

0,068 Mol Eisen(III)-chlorid werden in 20 ml absolutem Benzol zum Sieden erhitzt. Unter Rühren werden 0,0186 Mol 1-Brom-3-ethyl-adamantan(I), in 30 ml abs. Benzol gelöst, zugetopft. Danach wird 3h zum Sieden erhitzt. Das Reaktionsgemisch wird nach dem Abkühlen auf Eis/Salzsäure gegossen, die organische Phase abgetrennt und die wässrige Phase noch zwei Mal mit Benzol extrahiert. Die vereinigten organischen Phasen werden mit Wasser gewaschen, mit Calciumchlorid getrocknet, filtriert und im Vakuum eingeengt. Der Rückstand wird aus Methanol umkristallisiert. (Ausbeute 80%)

C. Darstellung von 1-Ethyl-3-hydroxy-5-phenyl-adamantan(III)

Zu einer Lösung aus 0,03 Mol Chromtrioxid in 20 ml Eisessig und 20 ml Essigsäureanhydrid werden bei 0 °C 0,0095 Mol 1-Ethyl-3-phenyl-adamantan(II) zugegeben und 24h bei 4 °C gerührt. Das Reaktionsgemisch wird dann auf Wasser gegossen und drei Mal mit Pentan extrahiert. Die organische Phase wird mit gesättigter Natriumchlorid-Lösung gewaschen, über Magnesiumsulfat getrocknet, filtriert und im Vakuum eingeengt. Der Rückstand wird mit 20 ml 2N NaOH und 50 ml Methanol hydrolysiert. Das Methanol wird anschliessend im Vakuum entfernt und der Rückstand mit Wasser verdünnt. Dann wird drei Mal mit Ether extrahiert. Die organische Phase wird getrocknet, filtriert und im Vakuum eingeengt. Der Rückstand wird aus Cyclohexan umkristallisiert. (Ausbeute 50%) Lit.: H.Stetter, M.Schwarz u. A.Hirschhorn, Chem. Ber. (1959), 92, 1629-35

D. Darstellung von 1-Brom-3-ethyl-5-phenyl-adamantan(IV)

0,03 Mol 1-Ethyl-3-hydroxy-5-phenyl-adamantan(III) werden mit 100 ml 40%iger HBr in Eisessig 20min. bei 60 ° C und 30 min bei Raumtemperatur gerührt. Das Reaktionsgemisch wird anschliessend mit Wasser verdünnt und mit Ether extrahiert. Die vereinigten organischen Extrakte werden mit Natriumchlorid-Lösung gewaschen, mit Magnesiumsulfat getrocknet, filtriert und im Vakuum eingeengt. Der Rückstand wird aus Methanol umkristallisiert. (Ausbeute 68%) Lit.: W.Fischer u. C.A.Grog, Helvetica Chim.Acta (1976), 59, 1953

E. Darstellung von 1-N-Formyl-3-ethyl-5-phenyladamantan(V)

0,03 Mol 1-Ethyl-3-hydroxy-5-phenyl-adamantan(IV) werden mit 50 ml Formamid 12h zum Rückfluss erhitzt. Nach dem Abkühlen giesst man das Reaktionsgemisch auf Wasser und extrahiert mit Dichlormethan. Die vereinigten organischen Phasen werden mit Magnesiumsulfat getrocknet, filtriert und eingeengt. (Ausbeute 80%)

F. Darstellung von 1-Amino-3-ethyl-5-phenyl-adamantan-Hydrochlorid(VI)

0,02 Mol 1-N-Formyl-3-ethyl-5-phenyl-adamantan(V) werden mit 100 ml 15%iger Salzsäure 24h zum Sieden erhitzt. Nach dem Abkühlen wird der Niederschlag abfiltriert und aus Isopropanol umkristallisiert. (Ausbeute 60%)

**Patentansprüche**

**1.** Verwendung von Adamantan-Derivaten der allgemeinen Formel

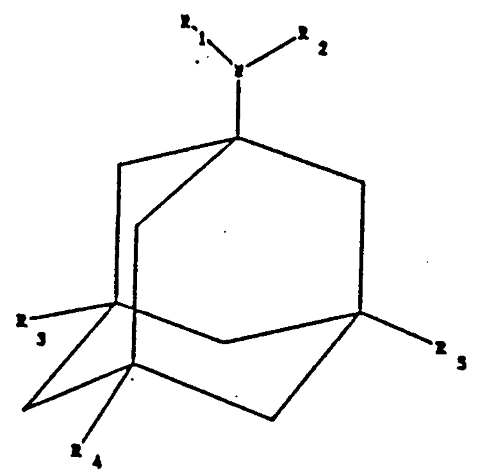

(I)

worin $R_1$ und $R_2$ gleich oder verschieden sind und Wasserstoff oder geradkettige oder verzweigte Alkylgruppen mit 1 bis 6 C-Atomen bedeuten oder zusammengenommen mit N eine heterocyclische Gruppe mit 5 oder 6 Ringgliedern darstellen,
$R_3$ und $R_4$ jeweils gleich oder verschieden sind und ausgewählt sind aus Wasserstoff, einem geradkettigen oder verzweigten Alkylrest mit 1 bis 6 C-Atomen, einem Cycloalkylrest mit 5 oder 6 C-Atomen, dem Phenylrest,
und worin $R_5$ Wasserstoff oder einen geradkettigen oder verzweigten $C_1$-$C_6$-Alkylrest darstellt,
sowie deren pharmazeutisch verträglichen Salze,
zur Herstellung eines Medikaments zur Behandlung der Schädigung von Hirnzellen infolge eine cerebralen Ischämie.

**2.** Verwendung gemäss Anspruch 1, wobei $R_1$, $R_2$ und $R_5$ Wasserstoff bedeuten.

**3.** Verwendung gemäss Anspruch 2, wobei $R_1$, $R_2$ und $R_5$ Wasserstoff und $R_3$ und $R_4$ Methyl bedeuten.

**4.** Verwendung gemäss Anspruch 2, wobei $R_1$, $R_2$ und $R_5$ Wasserstoff und $R_3$ und $R_4$ Ethyl bedeuten.

5. Verwendung gemäss Anspruch 1, wobei $R_1$, $R_2$, $R_4$ und $R_5$ Wasserstoff und $R_3$ Ethyl, Isopropyl oder Cyclohexyl bedeuten.

6. Verwendung gemäss Anspruch 1, wobei $R_2$ und $R_5$ Wasserstoff bedeuten.

7. Verwendung gemäss Anspruch 6, wobei $R_3$ und $R_4$ Methyl, $R_2$ und $R_5$ Wasserstoff und $R_1$ Methyl oder Ethyl bedeuten.

8. Verwendung gemäss Anspruch 1, worin $R_1$ und $R_2$ Wasserstoff bedeuten.

9. Verwendung gemäss Anspruch 8, worin $R_1$ und $R_2$ Wasserstoff,$R_3$ Ethyl und $R_5$ und $R_4$ Methyl bedeuten.

10. Verwendung gemäß Anspruch 1, wobei das Adamantanderivat 1-Amino-3-ethyl-5,7-dimethyl-adamantan ist.

11. Verwendung von Adamantan-Derivaten, wie sie in den Ansprüchen 1-9 offenbart werden, zur Herstellung eines Medikaments zur Behandlung von Morbus Alzheimer.

12. 1-Amino-3-cyclohexyl-adamantan.

**Claims**

1. The use of adamantane derivatives corresponding to the following general formula

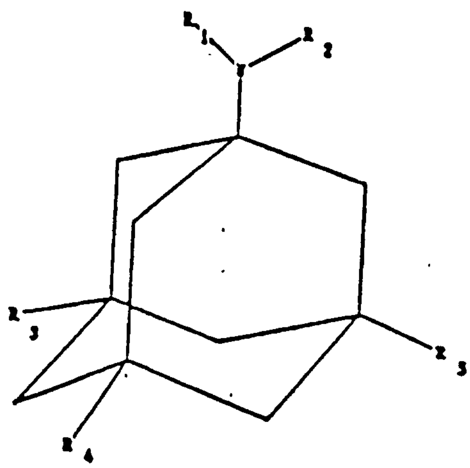

(I)

wherein $R_1$ and $R_2$ are identical or different and stand for hydrogen or straight chain or branched alkyl groups having 1 to 6 carbon atoms or together with N represent a heterocyclic group having 5 or 6 ring members,
$R_3$ and $R_4$ are identical or different and are selected from hydrogen, a straight chain or branched alkyl group having 1 to 6 carbon atoms, a cycloalkyl group having 5 or 6 carbon atoms or the phenyl group and
$R_5$ denotes hydrogen or a straight chain or branched $C_1$-$C_6$-alkyl group
and their pharmaceutically acceptable salts for the preparation of a medicament for the treatment of damage to brain cells due to cerebral ischaemia.

2. Use according to Claim 1 wherein $R_1$, $R_2$ and $R_5$ denote hydrogen.

3. Use according to Claim 2 wherein $R_1$, $R_2$ and $R_5$ denote hydrogen and $R_3$ and $R_4$ denote methyl.

4. Use according to Claim 2 wherein $R_1$, $R_2$ and $R_5$ denote hydrogen and $R_3$ and $R_4$ denote ethyl.

5. Use according to Claim 1 wherein $R_1$, $R_2$, $R_4$ and $R_5$ denote hydrogen and $R_3$ denotes ethyl, isopropyl or cyclohexyl.

6. Use according to Claim 1 wherein $R_2$ and $R_5$ denote hydrogen.

7. Use according to Claim 6 wherein $R_3$ and $R_4$ denote methyl, $R_2$ and $R_5$ denote hydrogen and $R_1$ denotes methyl or ethyl.

8. Use according to Claim 1 wherein $R_1$ and $R_2$ denote hydrogen.

9. Use according to Claim 8 wherein $R_1$ and $R_2$ denote hydrogen, $R_3$ denotes ethyl and $R_5$ and $R_4$ denote methyl.

10. Use according to Claim 1, wherein the adamantane derivative is 1-amino-3-ethyl-5,7-dimethyl-adamantane.

11. Use of adamantane derivatives as disclosed in claims 1 to 9 for the preparation of a medicament for the treatment of Alzheimer's disease.

12. 1-Amino-3-cyclohexyl-adamantane.

**Revendications**

1. Utilisation de dérivés d'adamantane de formule générale :

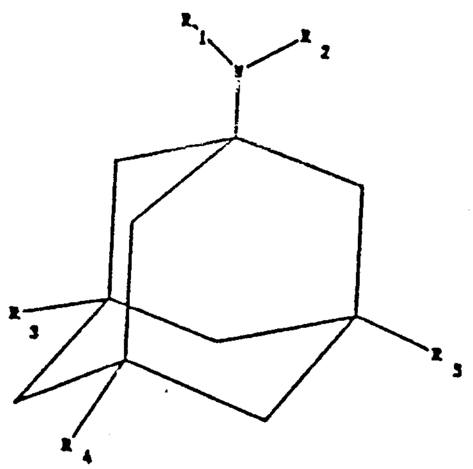

(I)

dans laquelle $R_1$ et $R_2$ sont identiques ou différents et représentent l'hydrogène ou des groupes alkyles à chaîne droite ou ramifiée en $C_1$-$C_6$ ou bien, pris avec l'atome d'azote, un groupe hétérocyclique à 5 ou 6 chaînons, $R_3$ et $R_4$ sont identiques ou différents et représentent chacun l'hydrogène, un reste alkyle à chaîne droite ou ramifiée en $C_1$-$C_6$, un groupe cycloalkyle en $C_5$ ou $C_6$, le reste phényle,
et dans laquelle $R_5$ représente l'hydrogène ou un reste alkyle à chaîne droite ou ramifiée en $C_1$-$C_6$.
et leurs sels acceptables en pharmacie, pour la préparation d'un médicament pour le traitement des lésions des cellules cérébrales résultant d'une ischémie cérébrale.

2. Utilisation selon la revendication 1, dans laquelle $R_1$, $R_2$ et $R_5$ représentent l'hydrogène.

3. Utilisation selon la revendication 2, dans laquelle $R_1$, $R_2$ et $R_5$ représentent l'hydrogène et $R_3$ et $R_4$ représentent des groupes méthyles.

4. Utilisation selon la revendication 2, dans laquelle $R_1$, $R_2$ et $R_5$ représentent l'hydrogène et $R_3$ et $R_4$ représentent des groupes éthyles.

5. Utilisation selon la revendication 1, dans laquelle $R_1$, $R_2$, $R_4$ et $R_5$ représentent l'hydrogène et $R_3$ représente un groupe éthyle, isopropyle ou cyclohexyle.

6. Utilisation selon la revendication 1, dans laquelle $R_2$ et $R_5$ représentent l'hydrogène.

7. Utilisation selon la revendication 6, dans laquelle $R_3$ et $R_4$ représenent des groupes méthyles, $R_2$ et $R_5$ représentent l'hydrogène et $R_1$ représente un groupe méthyle ou éthyle.

8. Utilisation selon la revendication 1, dans laquelle $R_1$ et $R_2$ représentent l'hydrogène.

9. Utilisation selon la revendication 8, dans laquelle $R_1$ et $R_2$ représentent l'hydrogène, $R_3$ représente un groupe éthyle et $R_5$ et $R_4$ représentent des groupes méthyles.

10. Utilisation selon la revendication 1, dans laquelle le dérivé d'adamantane est le 1-amino-3-éthyl-5,7-diméthyl-adamantane.

11. Utilisation de dérivés d'adamantane selon les revendications 1-9, pour la préparation d'un médicament pour le traitement de la maladie d'Alzheimer.

12. Le 1-amino-3-cyclohexyl-adamantane.

Abbildung 1

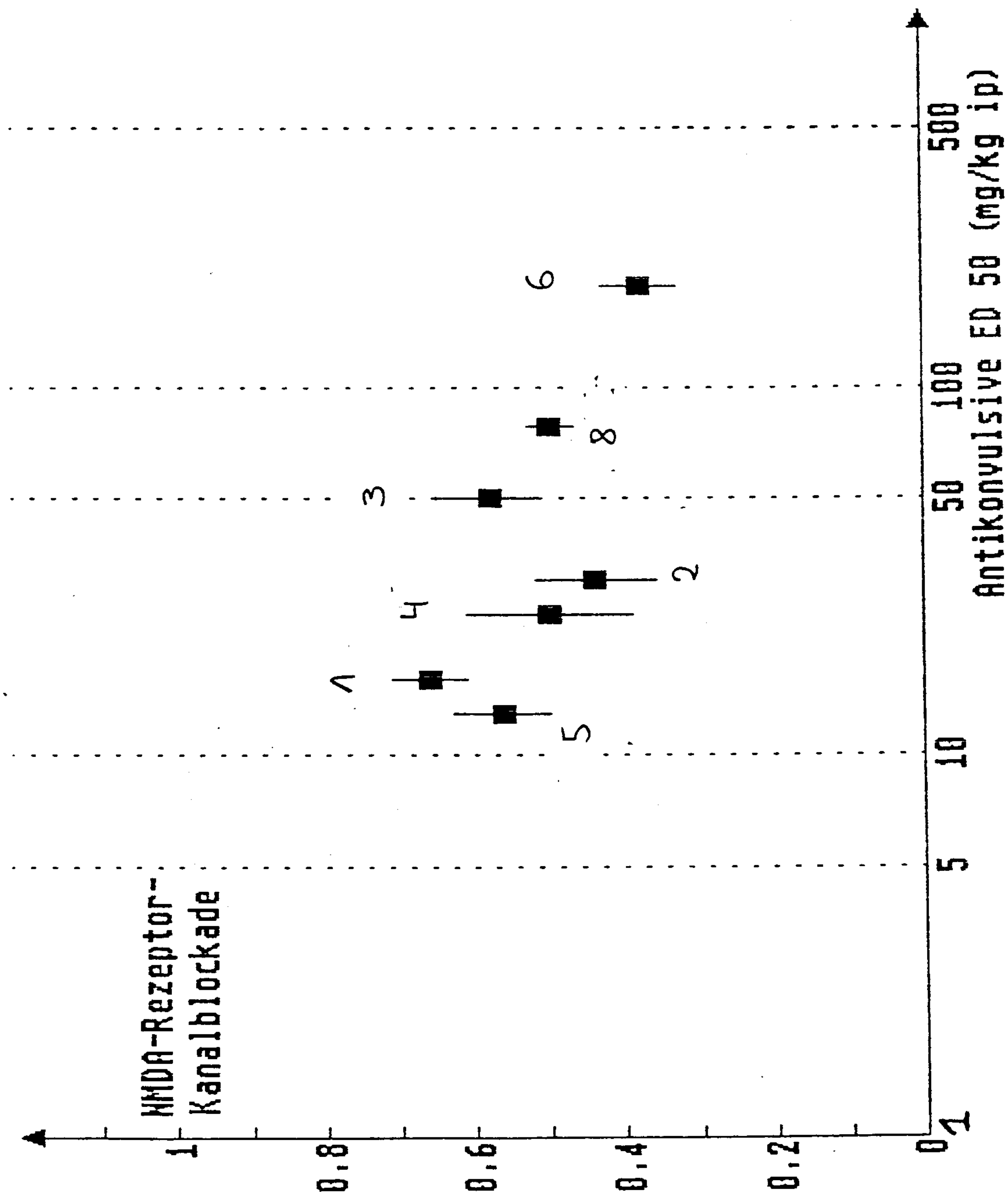
NMDA-Rezeptor-
Kanalblockade
Antikonvulsive ED 50 (mg/kg ip)
0
0,2
0,4
0,6
0,8
1
1
5
10
50
100
500
1
2
3
4
5
6
8